# FASCICULE DE BREVET EUROPEEN

(11) **EP 0 968 221 B1**
(45) Date de publication et mention de la délivrance du brevet: **02.05.2003**
(21) Numéro de dépôt: 98908141.9
(22) Date de dépôt: 06.02.1998
(51) Int. Cl.: C07H 3/04, C07H 13/04, C07H 15/203, A61K 7/48, A61K 7/00

(54) **NOUVEAUX DERIVES DE L'ACIDE SALICYLIQUE ET LEUR UTILISATION DANS LES COMPOSITIONS COSMETIQUES OU DERMATOLOGIQUES**
SALICYLSÄURE-DERIVATE UND IHRER VERWENDUNG IN KOSMETISCHE- ODER DERMATOLOGISCHE ZUSAMMENSETZUNGEN
NOVEL SALICYLIC ACID DERIVATIVES AND THEIR USE IN COSMETIC OR DERMATOLOGICAL COMPOSITIONS

(30) Priorité: 12.02.1997 FR 9701617
(43) Date de publication de la demande: 05.01.2000
(73) Titulaire: L'OREAL, 75008 Paris (FR)
(72) Inventeur: DALKO, Maria, F-91190 Gif sur Yvette (FR); GALEY, Jean-Baptiste, F-93600 Aulnay sous Bois (FR)
(74) Mandataire: Dodin, Catherine
(86) Numéro de dépôt international: FR9800231
(87) Numéro de publication internationale: WO98035973

(56) Documents cités:
- EP-A- 0 597 776
- EP-A- 0 649 648
- PATENT ABSTRACTS OF JAPAN vol. 097, no. 002, 28 février 1997 & JP 08 268872 A (SHISEIDO CO LTD), 15 octobre 1996,
- PATENT ABSTRACTS OF JAPAN vol. 097, no. 002, 28 février 1997 & JP 08 268871 A (SHISEIDO CO LTD), 15 octobre 1996,
- PATENT ABSTRACTS OF JAPAN vol. 097, no. 002, 28 février 1997 & JP 08 268870 A (SHISEIDO CO LTD), 15 octobre 1996,
- PATENT ABSTRACTS OF JAPAN vol. 097, no. 002, 28 février 1997 & JP 08 268869 A (SHISEIDO CO LTD), 15 octobre 1996,
- PATENT ABSTRACTS OF JAPAN vol. 097, no. 002, 28 février 1997 & JP 08 268868 A (SHISEIDO CO LTD), 15 octobre 1996,
- PATENT ABSTRACTS OF JAPAN vol. 097, no. 002, 28 février 1997 & JP 08 268858 A (SHISEIDO CO LTD), 15 octobre 1996,
- PATENT ABSTRACTS OF JAPAN vol. 008, no. 283 (C-258), 25 décembre 1984 & JP 59 152328 A (EISAI KK), 31 août 1984,

## Description

La présente invention se rapporte à de nouveaux dérivés de l'acide salicylique et à leur utilisation notamment dans les domaines cosmétique et/ou dermatologique. Ils peuvent être utilisés aussi dans le domaine pharmaceutique ou vétérinaire. Plus spécialement, ces dérivés sont utilisés dans une composition destinée au soin de la peau d'être humain, notamment pour lutter contre les signes du vieillissement cutané et/ou pour améliorer l'éclat du teint et/ou pour lisser la peau du visage et/ou du corps et/ou pour traiter les rides et les ridules de la peau et/ou pour stimuler le processus de renouvellement épidermique.

On cherche, de plus en plus, à paraître plus jeune et moins ridé, en utilisant des compositions cosmétiques contenant des actifs capables de lutter contre le vieillissement. Au cours du processus de vieillissement, il apparaît différents signes sur la peau, très caractéristiques de ce vieillissement, se traduisant notamment par une modification de la structure et des fonctions cutanées.

Les principaux signes cliniques du vieillissement cutané sont notamment l'apparition de ridules et de rides profondes, en augmentation avec l'âge. On constate en particulier une désorganisation du "grain" de la peau, c'est-à-dire que le micro-relief est moins régulier et présente un caractère anisotrope. Par ailleurs, le teint de la peau est généralement modifié, il apparaît plus pâle et plus jaune. On constate aussi une perte de fermeté et de tonicité de la peau.

On constate donc que les signes cliniques de vieillissement cutané résultent essentiellement d'un dysfonctionnement des principaux mécanismes biologiques intervenant au niveau de la peau.

Malheureusement, les actifs antivieillissement couramment utilisés tels que les rétinoïdes et les actifs acides comme les hydroxyacides, présentent l'inconvénient majeur de provoquer des picotements, des démangeaisons, des tiraillements après leur application, pouvant conduire à un inconfort ou une intolérance. L'utilisation de ces composés pour les utilisateurs à peau sensible est donc souvent rédhibitoire.

La demanderesse a donc cherché des actifs ayant le même effet que les actifs de l'art antérieur sans poser ces problèmes d'inconfort ou d'intolérance.

La demanderesse a découvert de façon surprenante de nouveaux dérivés de l'acide salicylique, appropriés pour le soin de la peau et permettant notamment de lutter contre les signes du vieillissement, d'améliorer l'éclat du teint de la peau, de lisser les traits de la peau et de stimuler le processus de renouvellement épidermique, sans provoquer d'irritation de la peau.

Il est connu du document EP-A-649648 des dérivés de l'acide hydroxysalicylique. Ces dérivés ont une structure différente des composés de la présente invention et présentent essentiellement des propriétés blanchissantes.

L'invention a pour objet un composé de formule générale (I) : 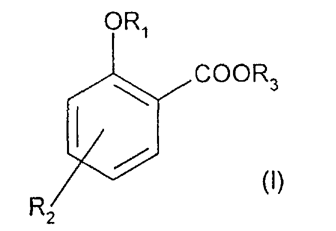 où R₁ représente l'hydrogène, un reste de mono- ou disaccharide, un groupement R₄-CO-, R₄ étant choisi parmi les groupements alkyle saturés en C₁ à C₂₀ linéaires, ramifiés ou cyclisés, ou les groupements alkyle insaturés en C₁ à C₂₀, les dits groupements alkyle étant éventuellement substitués ;
R₂ représente l'hydrogène ou un groupement R₅-CO-, R₅ étant choisi parmi les groupements alkyle en C₁ à C₂₀ linéaires ou ramifiés, saturés ou insaturés, éventuellement substitués ;
R₃ représente l'hydrogène, un groupement alkyle en C, à C₂₀ linéaire ou ramifié, saturé ou insaturé, éventuellement substitué, un reste de mono- ou disaccharide,
à la condition qu'au moins un des radicaux R₁ ou R₃ représente un reste de mono- ou disaccharide et que, quand R₂ est l'hydrogène, R₁ soit différent de -COCH₃.

Avantageusement, R₁ est l'hydrogène et R₃ représente un reste de mono- ou disaccharide, et en particulier un reste galactose.

R₂ peut être en position para ou ortho par rapport au groupe OR₁. De préférence, R₂ est en position para par rapport au groupe OR₁ et représente l'hydrogène ou un groupement R₅-CO- où R₅ est un groupe alkyle saturé comportant de 3 à 11 atomes de carbone. R₅ peut être notamment choisi parmi les groupes linéaires ou ramifiés, octyle, décyle, hexyle, dodécyle.

Comme substituants sur les groupes alkyle R₃, R₄, R₅, on peut citer les atomes d'halogène, le groupement trifluorométhyle, le groupement hydroxyle sous forme libre ou estérifiée par un acide ayant de 1 à 6 atomes de carbone ou bien les groupements carboxyle, libres ou estérifiés par un alcool inférieur ayant de 1 à 6 atomes de carbone.

De façon préférée, le composé selon l'invention est en particulier le 2-hydroxy benzoate de 3,4,5,6-tétrahydroxy-tétrahydropyran-2-ylméthyle, le 2-hydroxy-5-octanoyl-benzoate de 3,4,5,6-tétrahydroxy-tétrahydropyran-2-ylméthyle, le 2-hydroxy-5-octanoyl-benzoate de 2-(1,2-dihydroéthyl)-4,5-dihydroxy-tétrahydrofuran-3-yle.

Les composés de formule (I) sont nouveaux à l'exception de l'acide 2-O-β-D-glucopyranosyl salicylique et du glucose salicylate qui sont connus comme analgésiques pour traiter les maux d'estomac et les ulcères dans la demande de brevet JP59 152 328. Ils sont obtenus selon un procédé de synthèse classique. qui consiste à faire réagir le sucre protégé (par exemple sous forme d'acétonide ou par benzylation) et le chlorure d'acide du dérivé salicylé, ou à faire réagir le dérivé salicylé protégé sous forme d'ester et le chlorure d'acide du sucre protégé, puis à réaliser la déprotection du composé obtenu, par exemple en milieu acide dans le cas où le sucre était protégé par une fonction acétonide ou par hydrogénolyse dans le cas où le sucre était perbenzylé.

L'invention a aussi pour objet une composition comprenant au moins un composé tel que défini précédemment. Cette composition est en particulier une composition topique, en particulier cosmétique et/ou dermatologique. Dans ce cas, elle contient un milieu physiologiquement acceptable. Par milieu physiologiquement acceptable, on entend un milieu compatible avec la peau, les muqueuses, les ongles et/ou les cheveux.

Les composés selon l'invention peuvent être présents dans une composition selon l'invention en une quantité allant de 0,001 à 30 %, de préférence de 0,01 à 10 %, et mieux de 0,1 à 5 % du poids total de la composition.

Les compositions contenant un composé de formule (I) peuvent se présenter en particulier toutes les formes galéniques normalement appropriées pour une application topique, et par exemple sous forme d'une solution aqueuse, alcoolique, hydroalcoolique ou huileuse, d'un gel aqueux ou huileux, d'une composition solide anhydre, d'une dispersion du type lotion ou sérum, d'une émulsion du type eau-dans-huile (E/H), huile-dans-eau (H/E) ou multiple (E/H/E ou H/E/H), d'une microémulsion ou d'une dispersion de vésicules de type ionique et/ou non ionique. Ces compositions sont préparées selon les méthodes usuelles.

Ces compositions peuvent comprendre au moins un additif choisi parmi les corps gras, les conservateurs, les gélifiants, les parfums, les silicones, les tensioactifs, l'eau, les antioxydants, les charges, les filtres, les actifs physiologiquement acceptables tels que les hydratants, les vitamines, les actifs antivieillissement autres que les composés de formule (I), et leurs mélanges.

Les corps gras peuvent être choisis parmi les huiles telles que l'huile de jojoba, l'huile de vaseline, le palmitate d'isopropyle, les silicones (cyclopentadimethylsiloxane), les cires telles que la cire de sipol, la cire de carnauba, les acides gras, les alcools gras (alcool stéarylique) et leurs mélanges.

Les quantités des différents constituants des compositions selon l'invention sont celles classiquement utilisées dans les domaines considérés.

Ces compositions constituent notamment des crèmes de traitement ou de soin pour le visage, pour les mains ou pour le corps, (par exemple crèmes de jour, crèmes de nuit), des produits de maquillage comme des fonds de teint, des laits corporels de protection ou de soin, des lotions, gels ou mousses pour le soin de la peau.

L'invention a encore pour objet une utilisation du composé de formule (I) dans et/ou pour la fabrication d'une composition cosmétique et/ou dermatologique destinée au soin de la peau.

L'invention a aussi pour objet une utilisation du composé de formule (I) dans et/ou pour la fabrication d'une composition cosmétique et/ou dermatologique destinée à lutter contre les signes du vieillissement cutané et/ou à améliorer l'éclat du teint et/ou à lisser la peau du visage et/ou du corps et/ou à traiter les rides et les ridules de la peau et/ou à stimuler le processus de renouvellement épidermique.

L'invention a enfin pour objet un procédé de traitement cosmétique des signes du vieillissement cutané, qui consiste à appliquer sur la peau présentant ces signes le composé de formule (I) dans un milieu physiologiquement acceptable.

D'autres caractéristiques et avantages de l'invention ressortiront mieux des exemples qui suivent, donnés à titre illustratif et non limitatif.

Les exemples suivants 1 à 3 illustrent le procédé de préparation des dérivés conformes à l'invention.

### Exemple 1 : Préparation du 2-hydroxy benzoate de 3,4,5,6-tétrahydroxytétrahydro-pyran-2-ylméthyle

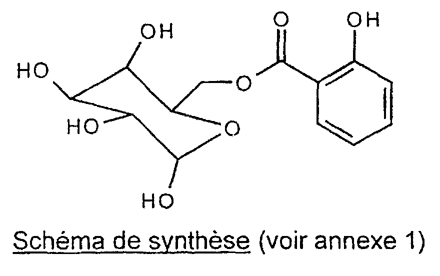

### Etape 1 : réaction du chlorure d'acide avec le galactose protégé

On additionne à 0°C sous argon une solution de 6 ml de dichlorométhane contenant 640 mg du chlorure d'acide acétylsalicylique à une solution de 10 ml de dichlorométhane contenant 700 mg de diisopropyl D-galactose et 1,5 ml de triéthylamine. La solution est agitée pendant 5 h à température ambiante. On additionne encore 240 mg de chlorure d'acide et 0,4 ml de triéthylamine dans 5 ml de dichlorométhane et on agite le mélange pendant 1 h. On ajoute ensuite 20 ml de NaHCO₃ saturé puis on sépare la phase organique et on la sèche sur sulfate de sodium. Le produit est purifié par chromatographie sur colonne de silice.

On obtient 0,9 g d'une huile incolore dont les spectres RMN ¹H, ¹³C et IR sont conformes à la structure attendue.

### Etape 2 : déprotection

On solubilise 105 mg du produit obtenu dans l'étape précédente, dans 10 ml d'un mélange acide trifluoroacétique / eau 1:1. La solution est agitée pendant 2 h 45 à 70°C puis évaporée à sec. Le résidu est purifié par chromatographie sur colonne de silice.

On obtient une huile incolore dont les spectres RMN ¹H et ¹³C sont conformes à la structure attendue.

Le rendement de la synthèse est de 52%.

| Analyse élémentaire : | | | |
|---|---|---|---|
| | C | H | O |
| Théorique | 52.0 | 5.37 | 42.63 |
| Trouvée | 51.64 | 5.32 | 42.39 |

### Exempte 2 : Préparation du 2-hydroxy-5-octanoyl-benzoate de 3,4,5,6-tétrahydroxy-tétrahydro-pyran-2-ylméthyle

Selon un schéma de synthèse analogue à celui de l'exemple 1, on réalise la synthèse du 2-hydroxy-5-octanoyl-benzoate de 3,4,5,6-tétrahydroxy-tétrahydropyran-2-ylméthyle de formule suivante :

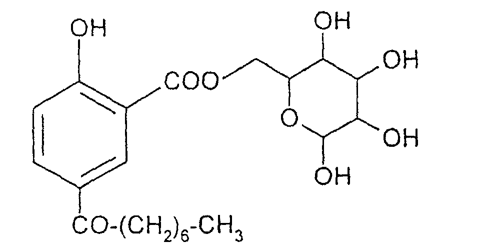

### Etape 1 : préparation du chlorure d'acide

A une solution de 5 g d'acide 2-acétoxy 5-octanoyl benzoïque dans 60 ml de toluène anhydre sous argon, on ajoute 16,3 ml d'une solution 2M de chlorure d'oxalyle dans le toluène. On additionne ensuite une goutte de DMF puis le milieu est agité 30 minutes à température ambiante. On évapore la solution et on obtient une huile jaune directement utilisée dans l'étape suivante.

### Etape 2 : réaction du chlorure d'acide avec le galactose protégé

On additionne à 0°C sous argon une solution de 50 ml de dichlorométhane contenant 7,9 g de diisopropyl D-galactose et 22,6 ml de triéthylamine à une solution de 7,9 g du chlorure d'acide obtenu dans la première étape dans 50 ml de dichlorométhane. La solution est agitée 60 h à température ambiante. On ajoute ensuite 100 ml de NaHCO₃ saturé puis la phase organique est séparée et séchée sur sulfate de sodium. Le produit est purifié par chromatographie sur colonne de silice.

On obtient 13,5 g d'un mélange contenant le produit attendu contaminé par le produit de désacétylation correspondant.

### Etape 3 : déprotection

1,06 g du produit obtenu dans l'étape précédente sont solubilisés dans 25 ml d'un mélange acide trifluoroacétique/eau 3:7. La solution est agitée 4h30 à 60°C puis évaporée à sec. Le résidu est repris dans 50 ml d'acétate d'éthyle et lavé à l'eau. Après purification par chromatographie sur colonne de silice, on obtient un solide blanc dont les spectres RMN ¹H et ¹³C sont conformes à la structure du 2-hydroxy-5-octanoyl-benzoate de 3,4,5,6-tétrahydroxy-tétrahydropyran-2-yl-méthyle.

| Analyse élémentaire : | | | |
|---|---|---|---|
| | C | H | O |
| Théorique | 59.14 | 7.09 | 33.76 |
| Trouvée | 58.38 | 7.06 | 34.12 |

### Exemple 3 : Préparation du 2-hydroxy-5-octanoyl-benzoate de 2-(1,2-dihydroéthyl)-4,5-dihydroxy-tétrahydro-furan-3-yle

Selon un schéma de synthèse analogue à celui de l'exemple 1, on réalise la synthèse du 2-hydroxy-5-octanoyl-benzoate de 2-(1,2-dihydroéthyl)-4,5-dihydroxy-tétrahydro-furan-3-yle en utilisant le 1,2,3,4-di-O-isopropylidène-D-galactopyranose. Les spectres RMN ¹H et ¹³C du produit obtenu sont conformes à la structure attendue.

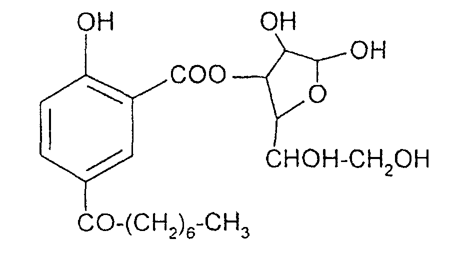

| Analyse élémentaire : | | | |
|---|---|---|---|
| | C | H | O |
| Théorique | 59.14 | 7.09 | 33.76 |
| Trouvée | 58.81 | 7.16 | 33.45 |

TEST : La demanderesse a constaté que la stimulation du processus de renouvellement épidermique consistant à éliminer les cellules superficielles de la peau entraînait un lissage des traits, un ravivage du teint et une réduction des rides et ridules. Aussi, la demanderesse a déterminé l'efficacité du traitement des signes du vieillissement des composés selon l'invention en effectuant un test *in vitro* de détachement cellulaire.

Ce test *in vitro* a été réalisé sur kératinocytes en utilisant les composés des exemples 2 et 3. Le principe du test repose sur le fait que le détachement cellulaire induit la libération de cornéocytes. Le pouvoir de traitement du vieillissement du produit testé va être d'autant plus grand que le nombre de cornéocytes libérés sera important.

Le protocole du test a été le suivant : à partir de biopsies de peau, on a obtenu, par séparation de l'épiderme, les kératinocytes que l'on a dissociés par action enzymatique à la trypsine et mis en culture à la concentration de 2.10⁻⁵ cellules/ml. La croissance et la différenciation des kératinocytes ont été obtenues par culture durant 10 à 20 jours en milieu spécifique.

Puis, après élimination du milieu de culture, on a ajouté le produit à tester et évalué l'activité du produit. Pour ce faire, on a réalisé deux prélèvements à T0 et T60, c'est-à-dire avant l'ajout du produit et 60 minutes après cet ajout, et on a analysé les prélèvements ainsi effectués au cytomètre de flux pour dénombrer la population de cornéocytes. Au cytomètre de flux, les populations de cornéocytes et de kératinocytes sont différenciées par traitement à l'acridine orange spécifique de l'ADN des cellules, qui se lie au noyau des cellules et révèle donc exclusivement la présence des kératinocytes.

L'indice de détachement cellulaire est déterminé par la différence entre T60 et T0.

La même mesure a été réalisée pour un témoin ne contenant pas de produit à tester car l'expérience produit inévitablement la libération de cornéocytes, même en absence d'actif. La variation du témoin a fixé arbitrairement la norme de 100%.

Les résultats sont rassemblés dans le tableau ci-dessous :

| | | |
|---|---|---|
| Témoin | Composé de l'exemple 2 5.10⁻⁵M | Composé de l'exemple 3 5.10⁻⁵M |
| 100% | 214.7% | 207.1 % |

Ces résultats montrent que les composés selon l'invention favorisent le détachement cellulaire, et permettent donc de lisser la peau et d'atténuer voire supprimer les signes du vieillissement.

Les exemples suivants illustrent les compositions cosmétiques ou dermatologiques selon l'invention. Les quantités sont données en pourcentage en poids.

### Exemple de formulation 1 : Crème de soin pour le visage

- composé de l'exemple 2 1 %
- stéarate de sodium (émulsionnant) 3 %
- huile de vaseline 6 %
- conservateur 0.05 %
- cyclopentadiméthylsiloxane 5 %
- alcool stéarylique 1 %
- parfum 1 %
- eau qsp 100 %

L'utilisation quotidienne de cette crème sur le visage permet de retrouver une peau plus lisse, plus jeune.

### Exemple de formulation 2 : Crème de soin pour le corps

- composé de l'exemple 1 1 %
- huile de jojoba 13 %
- palmitate d'isopropyle 2 %
- stéarate de polyéthylène glycol (émulsionnant) 3 %
- glycérol (hydratant) 15 %
- conservateur 0.5 %
- parfum 1 %
- eau qsp 100 %

On obtient une crème de soin qui agit contre le vieillissement sans irriter la peau.

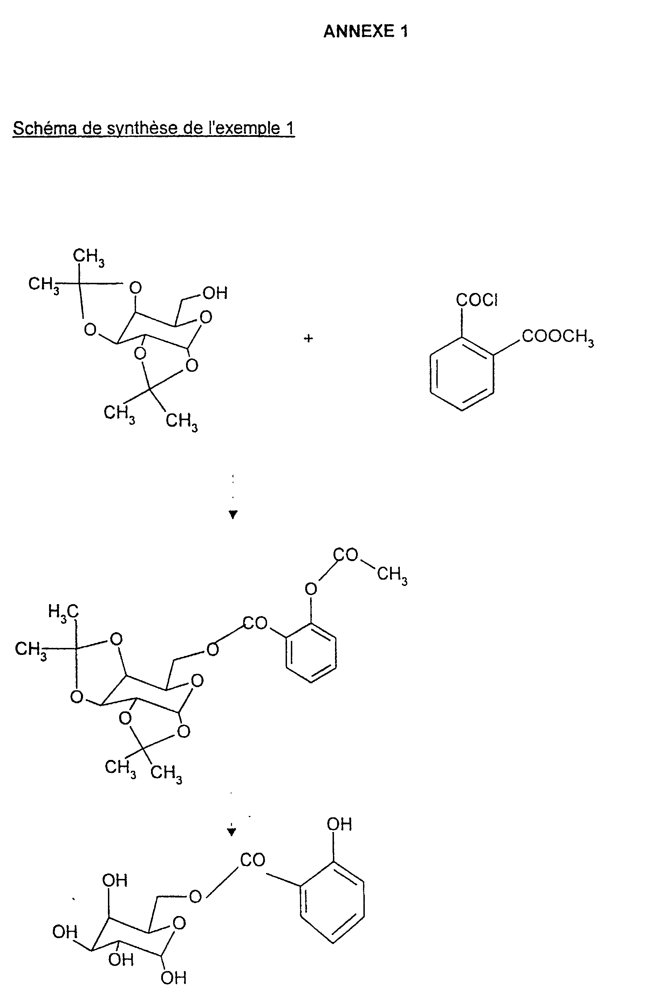

## Revendications

1. Composé de formule générale (I) : 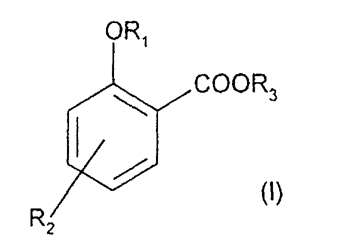 où R₁ représente l'hydrogène, un reste de mono- ou disaccharide, un groupement R₄-CO-, R₄ étant choisi parmi les groupements alkyle saturés en C₁ à C₂₀ linéaires, ramifiés ou cyclisés, ou les groupements alkyle insaturés en C₁ à C₂₀, les dits groupements alkyle étant éventuellement substitués ;
R₂ représente l'hydrogène ou un groupement R₅-CO-, R₅ étant choisi parmi les groupements alkyle en C₁ à C₂₀ linéaires ou ramifiés, saturés ou insaturés, éventuellement substitués ;
R₃ représente l'hydrogène, un groupement alkyle en C₁ à C₂₀ linéaire ou ramifié, saturé ou insaturé, éventuellement substitué, un reste de mono- ou disaccharide,
à la condition qu'au moins un des radicaux R₁ ou R₃ représente un reste de mono- ou disaccharide et que, quand R₂ est l'hydrogène, R₁ soit différent de -COCH₃ ; à l'exception de l'acide 2-O-β-D-glucopyranosyl salicylique et du glucose salicylate.

2. Composé selon la revendication 1, **caractérisé en ce que** R₁ est l'hydrogène.

3. Composé selon la revendication 1 ou 2, **caractérisé en ce que** R₂ est choisi parmi l'hydrogène et les groupements R₅-CO- où R₅ est un groupe alkyle saturé comportant de 3 à 11 atomes de carbone.

4. Composé selon l'une quelconque des revendications précédentes, **caractérisé en ce que** R₂ est choisi parmi les groupes linéaires ou ramifiés, octyle, décyle, hexyle, dodécyle.

5. Composé selon l'une quelconque des revendications précédentes, **caractérisé en ce que** R₃ représente un reste de mono- ou disaccharide.

6. Composé selon l'une quelconque des revendications précédentes, **caractérisé en ce qu'**il est choisi parmi le 2-hydroxy benzoate de 3,4,5,6-tétrahydroxytétrahydropyran-2-ylméthyle, le 2-hydroxy-5-octanoyl-benzoate de 3,4,5,6-tétrahydroxy-tétrahydro-pyran-2-ylméthyle, le 2-hydroxy-5-octanoyl-benzoate de 2-(1,2-dihydroéthyl)-4,5-dihydroxy-tétrahydro-furan-3-yle.

7. Composition **caractérisée en ce qu'**elle comprend au moins un composé tel que défini selon l'une quelconque des revendications 1 à 6.

8. Composition selon la revendication 7, **caractérisée en ce que** le composé est présent en une quantité allant de 0,001 à 30 % du poids total de la composition.

9. Composition selon la revendication 7 ou 8, **caractérisée en ce que** le composé est présent en une quantité allant de 0,01 à 10 % du poids total de la composition.

10. Composition selon l'une quelconque des revendications 7 à 9, **caractérisée en ce qu'**elle constitue une composition cosmétique et/ou dermatologique.

11. Composition selon l'une quelconque des revendications 7 à 10, **caractérisée en ce qu'**elle comprend de plus au moins un additif choisi parmi les corps gras, les conservateurs, les gélifiants, les parfums, les silicones, les tensioactifs, l'eau, les antioxydants, les charges, les filtres, les actifs physiologiquement acceptables et leurs mélanges.

12. Composition selon l'une quelconque des revendications 7 à 12, **caractérisée en ce qu'**elle se présente sous forme d'une solution, d'un gel, d'une dispersion, d'une émulsion, d'une microémulsion ou d'une dispersion de vésicules de type ionique et/ou non ionique.

13. Utilisation pour la fabrication d'une composition cosmétique ou dermatologique destinée à lutter contre les signes du vieillissement cutané et/ou à améliorer l'éclat du teint et/ou à lisser la peau du visage et/ou du corps et/ou à traiter les rides et les ridules de la peau et/ou à stimuler le processus de renouvellement épidermique, d'un composé de formule générale (I) : 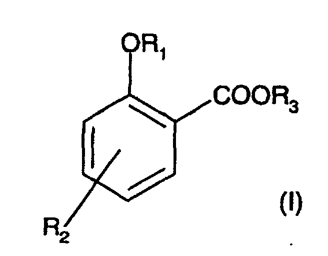 où R₁ représente l'hydrogène, un reste de mono- ou disaccharide, un groupement R₄-CO-, R₄ étant choisi parmi les groupements alkyle saturés en C1 à C20 linéaires, ramifiés ou cyclisés, ou les groupements alkyle insaturés en C1 à C20, les dits groupements alkyle étant éventuellement substitués ;
R₂ représente l'hydrogène ou un groupement R₅-CO-, R₅ étant choisi parmi les groupements alkyle en C1 à C20 linéaires ou ramifiés, saturés ou insaturés, éventuellement substitués ;
R₃ représente l'hydrogène, un groupement alkyle en C1 à C20 linéaire ou ramifié, saturé ou insaturé, éventuellement substitué, un reste de mono- ou disaccharide, à la condition qu'au moins un des radicaux R₁ ou R₃ représente un reste de mono- ou disaccharide et que, quand R₂ est l'hydrogène, R₁ soit différent de -COCH₃.

14. Procédé de traitement cosmétique des signes du vieillissement cutané, **caractérisé en ce qu'**il consiste à appliquer sur la peau présentant ces signes, un composé de formule (I) : 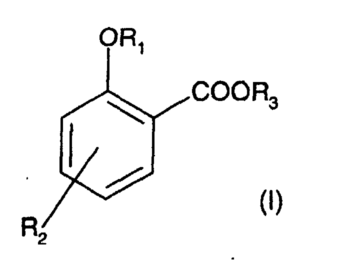 où R₁ représente l'hydrogène, un reste de mono- ou disaccharide, un groupement R₄-CO-, R₄ étant choisi parmi les groupements alkyle saturés en C1 à C20 linéaires, ramifiés ou cyclisés, ou les groupements alkyle insaturés en C1 à C20, les dits groupements alkyle étant éventuellement substitués ;
R₂ représente l'hydrogène ou un groupement R₅-CO-, R₅ étant choisi parmi les groupements alkyle en C1 à C20 linéaires ou ramifiés, saturés ou insaturés, éventuellement substitués ;
R₃ représenta l'hydrogène, un groupement alkyle en C1 à C20 linéaire ou ramifié, saturé ou insaturé, éventuellement substitué, un reste de mono- ou disaccharide, à la condition qu'au moins un des radicaux R₁ ou R₃ représente un reste de mono- ou disaccharide et que, quand R₂ est l'hydrogène, R₁ soit différent de -COCH₃,
dans un milieu physiologiquement acceptable.

## Patentansprüche

1. Verbindung der allgemeinen Formel (I): 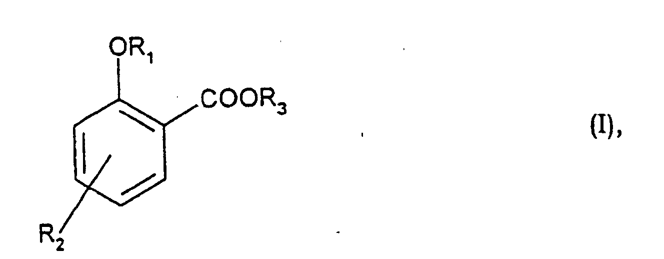 worin bedeuten:
- R₁ Wasserstoff, eine Mono- oder Disaccharidgruppe oder eine Gruppe R₄-CO-, wobei R₄ unter den geradkettigen, verzweigten oder cyclischen, gesättigten C₁₋₂₀-Alkylgruppen oder den ungesättigten C₁₋₂₀-Alkylgruppen ausgewählt ist, wobei die Alkylgruppen gegebenenfalls substituiert sein können;
- R₂ Wasserstoff oder eine Gruppe R₅-CO-, wobei R₅ unter den geradkettigen oder verzweigten, gesättigten oder ungesättigten C₁₋₂₀-Alkylgruppen ausgewählt ist, die gegebenenfalls substituiert sein können;
- R₃ Wasserstoff, eine geradkettige oder verzweigte, gesättigte oder ungesättigte C₁₋₂₀-Alkylgruppe, die gegebenenfalls substituiert ist, oder eine Mono- oder Disaccharidgruppe;
mit der Maßgabe, dass mindestens eine der Gruppen R₁ oder R₃ eine Mono- oder Disaccharidgruppe bedeutet, und dass R₁ von -COCH₃ verschieden ist, wenn R₂ Wasserstoff bedeutet,
wobei 2-O-β-D-Glucopyranosyl-L-salicylsäure und Glucosesalicylat ausgenommen sind.

2. Verbindung nach Anspruch 1, **dadurch gekennzeichnet, dass** R₁ Wasserstoff bedeutet.

3. Verbindung nach Anspruch 1 oder 2, **dadurch gekennzeichnet; dass** R₂ unter Wasserstoff und den Gruppen R₅-CO- ausgewählt ist,
wobei R₅ eine gesättigte Alkylgruppe mit 3 bis 11 Kohlenstoffatomen bedeutet.

4. Verbindung nach einem der vorhergehenden Ansprüche, **dadurch gekennzeichnet, dass** R₂ unter den geradkettigen oder verzweigten Gruppen Octyl, Decyl, Hexyl oder Dodecyl ausgewählt ist.

5. Verbindung nach einem der vorhergehenden Ansprüche, **dadurch gekennzeichnet, dass** R₃ eine Mono- oder Disaccharidgruppe bedeutet.

6. Verbindung nach einem der vorhergehenden Ansprüche, **dadurch gekennzeichnet, dass** sie unter 3,4,5,6-Tetrahydroxy-tetrahydropyran-2ylmethyl-2-hydroxy-benzoat, 3,4,5,6-Tetrahydroxy-tetrahydropyran-2ylmethyl-2-hydroxy-5-octanoyl-benzoat und 2-(1,2-Dihydroethyl)-4,5-dihydroxy-tetrahydrofuran-3yl-2-hydroxy-5-octanoyl-benzoat ausgewählt ist.

7. Zusammensetzung, **dadurch gekennzeichnet, dass** sie mindestens eine Verbindung nach einem der Ansprüche 1 bis 6 enthält.

8. Zusammensetzung nach Anspruch 7, **dadurch gekennzeichnet, dass** die Verbindung in einem Mengenanteil von 0,001 bis 30 % des Gesamtgewichts der Zusammensetzung vorliegt.

9. Zusammensetzung nach Anspruch 7 oder 8, **dadurch gekennzeichnet, dass** die Verbindung in einem Mengenanteil von 0,01 bis 10 % des Gesamtgewichts der Zusammensetzung vorliegt.

10. Zusammensetzung nach einem der Ansprüche 7 bis 9, **dadurch gekennzeichnet, dass** sie eine kosmetische und/oder dermatologische Zusammensetzung ist.

11. Zusammensetzung nach einem der Ansprüche 7 bis 10, **dadurch gekennzeichnet, dass** sie ferner einen Zusatzstoff enthält, der unter den Fettsubstanzen, Konservierungsmitteln, Gelbildnern, Parfums, Siliconen, grenzflächenaktiven Stoffen, Wasser, Antioxidantien, Füllstoffen, Filtern, physiologisch akzeptablen Wirkstoffen und deren Gemischen ausgewählt ist.

12. Zusammensetzung nach einem der Ansprüche 7 bis 11, **dadurch gekennzeichnet, dass** sie als Lösung, Gel, Dispersion, Emulsion, Mikroemulsion oder Vesikeldispersion vom ionischen und/oder nichtionischen Typ vorliegt.

13. Verwendung einer Verbindung der allgemeinen Formel (I) zur Herstellung einer kosmetischen oder dermatologischen Zusammensetzung, die dazu vorgesehen ist, die Anzeichen der Hautalterung zu bekämpfen und/oder den Teint strahlender erscheinen zu lassen und/oder die Haut des Gesichts und/oder des Körpers zu glätten und/oder die Falten und Fältchen der Haut zu behandeln und/oder den Prozess der Erneuerung der Epidermis zu stimulieren: 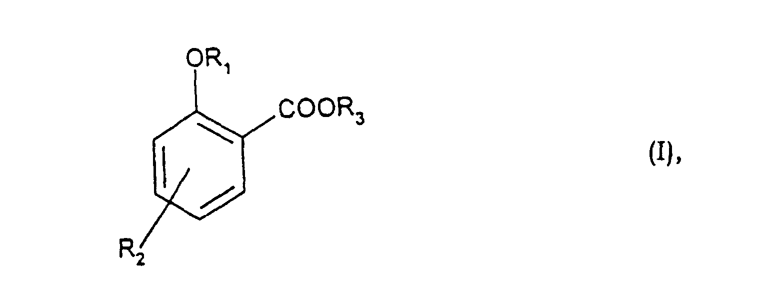 worin bedeuten:
- R₁ Wasserstoff, eine Mono- oder Disaccharidgruppe oder eine Gruppe R₄-CO-, wobei R₄ unter den geradkettigen, verzweigten oder cyclischen, gesättigten C₁₋₂₀-Alkylgruppen oder den ungesättigten C₁₋₂₀-Alkylgruppen ausgewählt ist, wobei die Alkylgruppen gegebenenfalls substituiert sein können;
- R₂ Wasserstoff oder eine Gruppe R₅-CO-, wobei R₅ unter den geradkettigen oder verzweigten, gesättigten oder ungesättigten C₁₋₂₀-Alkylgruppen ausgewählt ist, die gegebenenfalls substituiert sein können;
- R₃ Wasserstoff, eine geradkettige oder verzweigte, gesättigte oder ungesättigte C₁₋₂₀-Alkylgruppe, die gegebenenfalls substituiert ist, oder eine Mono- oder Disaccharidgruppe;
mit der Maßgäbe, dass mindestens eine der Gruppen R₁ oder R₃ eine Mono- oder Disaccharidgruppe bedeutet, und dass R₁ von -COCH₃ verschieden ist, wenn R₂ Wasserstoff bedeutet.

14. Verfahren zu kosmetischen Behandlung der Zeichen der Hautalterung, das darin besteht, auf die Haut, die solche Anzeichen zeigt, in einem physiologisch akzeptablen Medium eine Verbindung der allgemeinen Formel (I) aufzutragen: 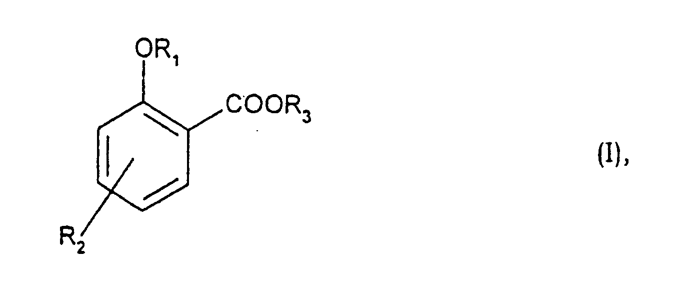 worin bedeuten:
- R₁ Wasserstoff, eine Mono- oder Disaccharidgruppe oder eine Gruppe R₄-CO-, wobei R₄ unter den geradkettigen, verzweigten oder cyclischen, gesättigten C₁₋₂₀-Alkylgruppen oder den ungesättigten C₁₋₂₀-Alkylgruppen ausgewählt ist, wobei die Alkylgruppen gegebenenfalls substituiert sein können;
- R₂ Wasserstoff oder eine Gruppe R₅-CO-, wobei R₅ unter den geradkettigen oder verzweigten, gesättigten oder ungesättigten C₁₋₂₀-Alkylgruppen ausgewählt ist, die gegebenenfalls substituiert sein können;
- R₃ Wasserstoff, eine geradkettige oder verzweigte, gesättigte oder ungesättigte C₁₋₂₀-Alkylgruppe, die gegebenenfalls substituiert ist, oder eine Mono- oder Disaccharidgruppe;
mit der Maßgabe, dass mindestens eine der Gruppen R₁ oder R₃ eine Mono- oder Disaccharidgruppe bedeutet, und dass R₁ von -COCH₃ verschieden ist, wenn R₂ Wasserstoff bedeutet.

## Claims

1. Compound of general formula (I): 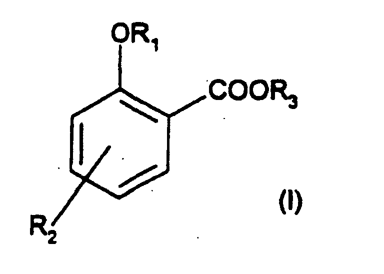 where R₁ represents hydrogen, a mono- or disaccharide residue, a group R₄-CO-, R₄ being chosen from linear, branched or cyclic C₁ to C₂₀ saturated alkyl groups or C₁ to C₂₀ unsaturated alkyl groups, the said alkyl groups optionally being substituted;
R₂ represents hydrogen or a group R₅-CO-, R₅ being chosen from linear or branched, saturated or unsaturated, optionally substituted C₁ to C₂₀ alkyl groups;
R₃ represents hydrogen, a linear or branched, saturated or unsaturated, optionally substituted C₁ to C₂₀ alkyl group, or a mono- or disaccharide residue, on condition that at least one of the radicals R₁ or R₃ represents a mono- or disaccharide residue and that, when R₂ is hydrogen, R₁ is other than -COCH₃; except for 2-O-β-D-glucopyranosyl salicylic acid and glucose salicylate.

2. Compound according to Claim 1, **characterized in that** R₁ is hydrogen.

3. Compound according to Claim 1 or 2, **characterized in that** R₂ is chosen from hydrogen and groups R₅-CO-where R₅ is a saturated alkyl group containing from 3 to 11 carbon atoms.

4. Compound according to any one of the preceding claims, **characterized in that** R₂ is chosen from linear or branched octyl, decyl, hexyl and dodecyl groups.

5. Compound according to any one of the preceding claims, **characterized in that** R₃ represents a mono- or disaccharide residue.

6. Compound according to any one of the preceding claims, **characterized in that** it is chosen from 3,4,5,6-tetrahydroxytetrahydropyran-2-ylmethyl 2-hydroxybenzoate, 3,4,5,6-tetrahydroxytetrahydropyran-2-ylmethyl 2-hydroxy-5-octanoylbenzoate or 2-(1,2-dihydroethyl)-4,5-dihydroxytetrahydrofuran-3-yl 2-hydroxy-5-octanoylbenzoate.

7. Composition, **characterized in that** it comprises at least one compound as defined according to any one of Claims 1 to 6.

8. Composition according to Claim 7, **characterized in that** the compound is present in an amount ranging from 0.001 to 30% relative to the total weight of the composition.

9. Composition according to Claim 7 or 8, **characterized in that** the compound is present in an amount ranging from 0.01 to 10% relative to the total weight of the composition.

10. Composition according to any one of Claims 7 to 9, **characterized in that** it constitutes a cosmetic and/or dermatological composition.

11. Composition according to any one of Claims 7 to 10, **characterized in that** it also comprises at least one additive chosen from fatty substances, preserving agents, gelling agents, fragrances, silicones, surfactants, water, antioxidants, fillers, screening agents, physiologically acceptable active agents, and mixtures thereof.

12. Composition according to any one of Claims 7 to 12, **characterized in that** it is in the form of a solution, a gel, a dispersion, an emulsion, a microemulsion or a dispersion of vesicles of ionic and/or nonionic type.

13. Use, for the manufacture of a cosmetic or dermatological composition intended to combat the signs of ageing of the skin and/or to improve the radiance of the complexion and/or to smooth out facial and/or body skin and/or to treat wrinkles and fine lines on the skin and/or to stimulate the process of epidermal renewal, of a compound of general formula (I): 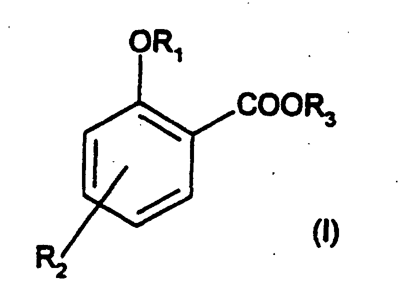 where R₁ represents hydrogen, a mono- or disaccharide residue, a group R₄-CO-, R₄ being chosen from linear, branched or cyclic C₁ to C₂₀ saturated alkyl groups or C₁ to C₂₀ unsaturated alkyl groups, the said alkyl groups optionally being substituted;
R₂ represents hydrogen or a group R₅-CO-, R₅ being chosen from linear or branched, saturated or unsaturated, optionally substituted C₁ to C₂₀ alkyl groups;
R₃ represents hydrogen, a linear or branched, saturated or unsaturated, optionally substituted C₁ to C₂₀ alkyl group, or a mono- or disaccharide residue, on condition that at least one of the radicals R₁ or R₃ represents a mono- or disaccharide residue and that, when R₂ is hydrogen, R₁ is other than -COCH₃.

14. Process for cosmetic treatment of the signs of ageing of the skin, **characterized in that** it consists in applying, to skin showing these signs, a compound of formula (I): 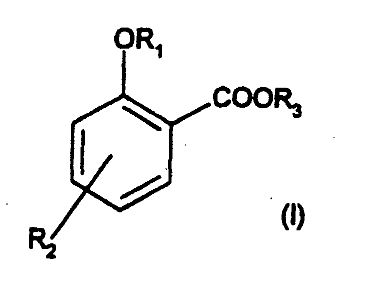 where R₁ represents hydrogen, a mono- or disaccharide residue, a group R₄-CO-, R₄ being chosen from linear, branched or cyclic C₁ to C₂₀ saturated alkyl groups or C₁ to C₂₀ unsaturated alkyl groups, the said alkyl groups optionally being substituted;
R₂ represents hydrogen or a group R₅-CO-, R₅ being chosen from linear or branched, saturated or unsaturated, optionally substituted C₁ to C₂₀ alkyl groups;
R₃ represents hydrogen, a linear or branched, saturated or unsaturated, optionally substituted C₁ to C₂₀ alkyl group, or a mono- or disaccharide residue, on condition that at least one of the radicals R₁ or R₃ represents a mono- or disaccharide residue and that, when R₂ is hydrogen, R₁ is other than -COCH₃,
in a physiologically acceptable medium.
